(19) Europäisches Patentamt
European Patent Office
Office européen des brevets

(11) Publication number: **0 457 966 A1**

# (12) EUROPEAN PATENT APPLICATION

(21) Application number: **90124902.9**

(51) Int. Cl.[5]: **C07C 211/63**, C07C 209/00

(22) Date of filing: **20.12.90**

(30) Priority: **21.05.90 US 525577**

(43) Date of publication of application:
**27.11.91 Bulletin 91/48**

(84) Designated Contracting States:
**BE CH DE DK FR GB IT LI NL SE**

(71) Applicant: **ROCKWELL INTERNATIONAL CORPORATION**
**2230 East Imperial Highway**
**El Segundo California 90245(US)**

(72) Inventor: **Christe, Karl Otto**
**5645 Parkmor Road**
**Calabasas, California 91302(US)**
Inventor: **Wilson, William Wayne**
**2317 Kentland**
**Simi Valley, California 93065(US)**

(74) Representative: **Wächtershäuser, Günter, Dr.**
**Tal 29**
**W-8000 München 2(DE)**

## (54) Anhydrous, chloride-and bifluoride-free tetramethylammonium fluoride.

(57) A method is disclosed for the preparation of tetramethylammonium fluoride having a water content of less than 0.1 weight percent.

EP 0 457 966 A1

## Statement of Government Interest

The Government has rights in this invention pursuant to Contract No. F04611-86-C-0074 awarded by the U.S. Department of the Air Force.

## Background of the Invention

### 1. Field of the Invention

This invention relates to tetramethylammonium fluoride as a free fluoride ion source which is highly soluble in anhydrous organic solvents. The free fluoride anion is a very strong Lewis base and plays an important role in many organic and inorganic reactions. For example, it is a very useful polymerization catalyst and a halogen exchange reagent in organic molecules.

### 2. Description of Background Art

Although alkali metal fluorides are readily available, they are practically insoluble in common organic solvents and, therefore, are of very limited practical use. This solubility problem can be overcome by the use of tetraalkylammonium fluorides. However, based on the prior art, these materials could not be prepared in the necessary pure form, i.e. anhydrous and free from chloride and bifluoride impurities. Thus, the tetraalkylammonium fluorides are generally available only as hydrates which, upon attempted water removal, undergo an $E_2$ elimination reaction with the formation of bifluoride and an olefin. This has prompted Sharma and Fry (J. Org. Chem. 1983, 48, 2112) to conclude that "it is very unlikely that pure, anhydrous tetraalkylammonium fluoride salts have ever, in fact, been produced in the case of ammonium ions susceptible to $E_2$ elimination; rather, reactions which have been reported to proceed in the presence of naked fluoride ion generated from such sources have probably actually been caused either by hydrated fluoride ion or by bifluoride ion." A similar conclusion has recently been reached for tetramethylammonium fluoride by Emsley (Polyhedron, 1985, 4, 489) who stated that "$N(CH_3)_4F$ has never been obtained anhydrous and that removal of water results in decomposition." In view of these reports, it is not surprising that $N(CH_3)_4F$ has not been exploited as a readily accessible, chemically inert, and highly soluble form of naked fluoride, and that costly alternatives, such as tris(dimethylamino)sulfonium difluorotrimethylsilicate, $[(CH_3)_2N]_3S^+F_2Si(CH_3)_3$, have been developed, (see W.J. Middleton, U.S. Patent 3,940,402). Although the latter compound does not contain a free fluoride ion per se, the $F_2Si(CH_3)_3$ anion serves as an excellent fluoride ion donor toward stronger Lewis acids.

Numerous reports on the synthesis of tetramethylammonium fluoride can be found in the literature. They are based on two approaches. The first one dates back to 1888, (Lawson and Collie, J. Chem. Soc., 1888, 53, 624), and involves the neutralization of $N(CH_3)_4OH$ with HF in aqueous solution, followed by water removal in vacuo at temperatures up to 160 °C. If the water is removed at various temperatures, intermediate $N(CH_3)_4F \cdot nH_2O$ type hydrates can be isolated with n ranging from 1 to 5. The difficulty with this process is the removal of the last amounts of water from the $N(CH_3)_4F$, because at 160 °C the removal rate is still slow while the $N(CH_3)_4F$ already begins to undergo a very slow decomposition. Thus, the products obtained by this method have been reported to contain significant amounts of impurities, attributed to either $HF_2$, (Tunder and Siegel, J. Inorg. Nucl. Chem., 1963, 25, 1097) or the monohydrate, (Harmon et al., J. Phys. Chem., 1974, 78, 2585). In a minor modification, (Klanberg and Muetterties, Inorg. Chem., 1968, 7, 155), of this method, the bulk of the water was removed from the aqueous $N(CH_3)_4F$ solution in vacuo on a rotating evaporator. The resulting syrupy oils were converted to a $N(CH_3)_4F \cdot CH_3OH$ solvate by repeated treatment with $CH_3OH$ and drying in vacuo at 100 °C.

The second approach utilizes metathetical reactions, such as

$$N(CH_3)_4Cl + KF \xrightarrow{CH_3OH} N(CH_3)_4F + KCl\downarrow .$$

The resulting crude $N(CH_3)_4F$ was purified by recrystallization from isopropanol, (Tunder and Siegel, 1963). This synthetic method has frequently been used, but, according to Klanberg and Muetterties, the $N(CH_3)_4F$ prepared in this manner always contained at least 1-2% of chloride, while commercially available $N(CH_3)_4F$ had an even higher $Cl^-$ content of 6.6%. Another metathesis that has been described in a German patent (Urban and Dotzer, German Patent 1,191,813) is based on the reaction of $N(CH_3)_4Cl$ with HF and $NaOCH_3$

in $CH_3OH$ to give $N(CH_3)_4F \cdot 2CH_3OH$. This solvate is then converted to $N(CH_3)_4F$ by vacuum pyrolysis at 150°C. The reaction of $N(CH_3)_4Cl$ with HF and $NaOCH_3$ must be carried out in two separate steps. Reacting the $N(CH_3)_4Cl$ first with $NaOCH_3$ according to

$$N(CH_3)_4Cl + NaOCH_3 \xrightarrow{CH_3OH} N(CH_3)_4OCH_3 + NaCl\downarrow$$

$$N(CH_3)_4OCH_3 + HF + CH_3OH \xrightarrow{CH_3OH} N(CH_3)_4F \cdot 2CH_3OH$$

results in a more efficient use of the starting materials than the reverse sequence

$$N(CH_3)_4Cl + 10HF \rightarrow N(CH_3)_4H_2F_3 + 7HF + HCl$$

$$N(CH_3)_4H_2F_3 + 2NaOCH_3 \xrightarrow{CH_3OH} N(CH_3)_4F \cdot 2CH_3OH + 2NaF\downarrow.$$

According to Klanberg and Muetterties these $NaOCH_3$-HF based processes suffer from the same drawback, i.e. $Cl^-$ impurities, as the $N(CH_3)_4Cl$ + KF metathesis, and the use of excess HF will generate $HF_2$ impurities.

In summary, although tetramethylammonium fluoride had been known for more than 100 years, pure anhydrous tetramethylammonium fluoride had previously not been available, and most of the proper- ties previously reported for this compound were due to either the bifluoride or monohydrate.

## Objects of the Invention

It is, therefore, an object of this invention to provide pure, anhydrous tetramethylammonium fluoride.

Another object is to provide a relatively simple, low-cost process for preparing such a material.

Other object advantages and novel features of the present invention will become apparent from the following detailed descrip- tion.

## Summary of Invention

Accordingly, the present invention provides pure and anhydrous tetramethylammonium fluoride and a simple method of producing the same.

## Detailed Description of the Invention

According to the present invention, pure, anhydrous and chloride, bifluoride and bicarbonate free tetramethylammonium fluoride can be prepared by a process involving the following steps:

(1) Neutralizing an aqueous tetramethylammonium fluoride hydroxide solution with an aqueous HF solution according to

$$N(CH_3)_4OH + HF \xrightarrow{H_2O} N(CH_3)_4F + H_2O.$$

When carrying out this reaction, it is important to maintain the following reaction conditions:

a) The handling and neutralization of the $N(CH_3)_4OH$ solution must be done in a $CO_2$ free atmosphere to avoid the formation of bicarbonate impurities.

b) Only plastic equipment must be used in the neutralization step, and contact with glass must be avoided to prevent the formation of $SiF_6^{2-}$, which can be formed by the attack of HF on glass.

c) The $N(CH_3)_4OH$ must be titrated exactly to the equivalence point of aqueous $N(CH_3)_4F$. The exact pH value of the solution (equivalence or endpoint) can be readily monitored by a pH electrode. Use of an excess of HF will result in the formation of a bifluoride impurity.

(2) Removing the water from the system in a dynamic vacuum at 155°C using a rotary film evaporator.

Precise temperature control (±5°C) is important for this step. Water removal is essentially complete when a constant weight is reached and the infrared absorption bands of the monohydrate, $N(CH_3)_4F\cdot H_2O$, at 822 and 895 $cm^{-1}$ show an intensity comparable to or less than that of the weak $N(CH_3)_4$ band at 1203 $cm^{-1}$.

(3) Removing residual water from the system by an azeotropic distillation using dry isopropanol.

(4) Removing the last traces of water by recrystallizing the crude $N(CH_3)_4F$ from dry isopropanol having a water content of less than 0.03%.

The yield of the purified $N(CH_3)_4F$ obtained in this manner is in excess of 80%, and material with a water content of less than 0.06 weight % was obtained by this method. The purity of the material was verified by Karl Fischer titration, infrared, Raman, $^1H$ and $^{19}F$ nuclear magnetic resonance spectroscopy, powder x-ray diffractometry, and vacuum pyrolysis at 230°C which produced pure $N(CH_3)_3$ and $CH_3F$ in quantitative yield.

The properties of pure $N(CH_3)_4F$ (see Tables 1-3) differ significantly from those previously reported, hereby confirming the conclusion of Sharma and Fry, Emsley and Klanberg and Muetterties that pure $N(CH_3)_4F$ had previously never been obtained.

Table 1. X-Ray Powder Data[a] for Pure Anhydrous $N(CH_3)_4F$

| d(obsd),Å | d(calcd),Å | Intensity | h | k | l |
|---|---|---|---|---|---|
| 5.35 | 5.356 | vs | 1 | 0 | 0 |
| 4.57 | 4.577 | s | 1 | 0 | 1 |
| 4.40 | 4.406 | ms | 0 | 0 | 2 |
| 3.398 | 3.403 | mw | 1 | 0 | 2 |
| 3.092 | 3.092 | m | 1 | 1 | 0 |
| 2.677 | 2.678 | m | 2 | 0 | 0 |
| 2.565 | 2.562 | ms | 2 | 0 | 1 |
| 2.530 | 2.531 | ms | 1 | 1 | 2 |
| 2.290 | 2.289 | mw | 2 | 0 | 2 |
| 2.202 | 2.203 | w | 0 | 0 | 4 |
| 1.979 | 1.979 | m | 2 | 0 | 3 |
| 1.838 | 1.839 | mw | 2 | 1 | 2 |
| 1.794 | 1.794 | w | 1 | 1 | 4 |
| 1.751 | 1.750 | w | 3 | 0 | 1 |
| 1.667 | 1.667 | mw | 2 | 1 | 3 |

a Hexagonal; a = 6.185Å, c = 8.812Å; Cu Kα radiation, Ni Filter.

Table 2. Vibrational Spectra of Pure Anhydrous Solid $N(CH_3)_4F$ and Their Assignments

| obsd freq, $cm^{-1}$ (rel intens) IR | Raman | assignment in point group $T_d$ |
|---|---|---|
| 3473 w, br | | $\nu_{CH_3} + \nu_{19}$ |
| 3376 w, br | | $\nu_{CH_3} + \nu_8$ |
| 3030 sh | 3029 sh | $\nu_{CH_3}$ and binary bands |
| 3008 | 3007 (2) | |
| 2980 | 2978 (1.3) | |
| 2928 s, br | | |
| | 2889 (1.9) | |
| 2822 | 2822 (1.4) | |
| 2785 | | |
| 2625 w | | $\nu_7 + \nu_{16}$, $2\nu_{17}$ |
| 2568 w | | $\nu_3 + \nu_8 + \nu_{16}$ |
| 2522 w | | $\nu_7 + \nu_{17}$ |
| 2392 w | | $\nu_{16} + \nu_{18}$ |
| 1880 w, vbr | | $\nu_8 + \nu_{15}$ |
| 1770 w, br | | $\nu_{17} + \nu_{19}$ |
| 1512 s | 1512 (0.4) | $\nu_{15}$ |
| 1490 sh | 1490 sh | |

| | | |
|---|---|---|
| | 1479 (2.9) | $\nu_6$ |
| | 1467 sh | $\nu_2$ |
| 1423 m | 1424 (0.7) | $\nu_{16}$ |
| 1415 sh | 1415 sh | |
| 1312 vvw | 1314 (0.4) | $\nu_{17}$ |
| 1209 w | 1209 (1.3) | $\nu_7$ |
| 1094 vw | | 3 x 367 or $\nu_{11}$ |
| 970 vs | 969 (5.8) | $\nu_{18}$ |
| 955 sh | | |
| 934 sh | 930 (0.2) | $2\nu_{19}$ |
| 767 vw | 767 (10) | $\nu_3$ |
| 466 ms | 467 (2.4) | $\nu_{19}$ |
| | 387 (0.4) | $\nu_8$ |
| 367 w | 368 (0.3) | $\nu_8$ or $\nu_{12}$ |

Table 3. $^{19}F$ NMR Spectra of Pure $N(CH_3)_4F$ in Different Solvents

| Solvent | Chemical Shift (ppm, upfield from $CFCl_3$) |
|---|---|
| $(CH_3)_2SO$ | 73 |
| $CH_3CN$ | 74 |
| $H_2NCHO$ | 96 |
| $CH_2Cl_2$ | 97 |
| $CH_3COCH_3$ | 103 |
| $CHCl_3$ | 113 |
| $H_2O$ | 119 |
| $(CH_3)_2CHOH$ | 122 |
| $CH_3CH_2OH$ | 137 |
| $CH_3OH$ | 148 |

Various modifications may be made to the present invention without departing from the spirit of the invention or the scope of the appended claims.

## Claims

1. Tetramethylammonium fluoride having a water content of less than 0.1 weight percent.

2. Tetramethylammonium fluoride having a water content of less than 0.1 weight percent and no detectable $Cl^-$, $HF_2$ and $HCO_3$ impurities.

3. A process of making the compounds of claims 1 and 2, comprising:
   a) neutralizing aqueous $N(CH_3)_4OH$ with aqueous HF to the exact equivalence point of aqueous $N(CH_3)_4F$;
   b) removing water from the aqueous $N(CH_3)_4F$ in a dynamic vacuum of about 155° C in a rotary film evaporator;
   c) removing residual water from the aqueous $N(CH_3)_4F$ by azeotropic distillation; and
   d) recrystallizing $N(CH_3)_4F$.

4. The process of claim 3 wherein the neutralization of aqueous $N(CH_3)_4OH$ is carried out in a $CO_2$ free atmosphere.

5. The process of claim 3 wherein the neutralization of aqueous $N(CH_3)_4OH$ is conducted utilizing plastic equipment and a pH electrode for control of the equivalence point.

6. The process of claim 3 wherein the water removal of step (b) is maintained until a constant weight is reached and the infrared absorptions due to the monohydrate $N(CH_3)_4F{\cdot}H_2O$ show an intensity comparable to that of the weak $N(CH_3)_4$ band at 1203 $cm^{-1}$.

7. The process of claim 3 wherein the azeotropic distillation utilizes dry isopropanol as solvent.

European Patent Office

# EUROPEAN SEARCH REPORT

Application Number

**EP 90 12 4902**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | DE-B-1 191 813 (SIEMENS-SCHUCKERTWERKE AG.) * the whole document * | 1 | C 07 C 211/63<br>C 07 C 209/00 |
| A,D | US-A-3 940 402 (WILLIAM JOSEPH MIDDLETON ET AL.) * the whole document * | 1 | |
| A,D | JOURNAL OF ORGANIC CHEMISTRY. vol. 48, no. 12, 17 June 1983, EASTON US pages 2112 - 2114; RAMESH K. SHARMA ET AL.: "INSTABILITY OF ANHYDROUS TETRA-N-ALKYLAMMONIUM FLUORIDES" * the whole document * | 1 | |
| A,D | JOURNAL OF THE CHEMICAL SOCIETY. vol. 53, 1888, LETCHWORTH GB pages 624 - 636; A. T. LAWSON ET AL.: "XLVII. THE ACTION OF HEAT ON THE SALTS OF TETRAMETHYLAMMONIUM" * pages 626 - 628 * | 1 | |
| A,D | POLYHEDRON. vol. 4, no. 3, 1985, OXFORD GB pages 489 - 490; JOHN EMSLEY: "LATTICE ENERGIES OF THE ALKALI METAL BIFLUORIDES, U[MHF2]" * the whole document * | 1 | **TECHNICAL FIELDS SEARCHED (Int. Cl.5)** |
| A,D | JOURNAL OF INORGANIC AND NUCLEAR CHEMISTRY. vol. 25, no. 9, September 1963, OXFORD GB pages 1097 - 1098; R. TUNDER ET AL.: "THE SF5 ANION" * page 1098 * | 1 | C 07 C |
| A,D | JOURNAL OF PHYSICAL CHEMISTRY. vol. 78, no. 25, 05 December 1974, EASTON US pages 2585 - 2591; KENNETH M. HARMON ET AL.: "HYDROGEN BONDING. IV. CORRELATION OF INFRARED SPECTRAL PROPERTIES WITH C-H...X . HYDROGEN BONDING AND CRYSTAL HABIT IN TETRAMETHYLAMMONIUM ION SALTS " * the whole document * | | |

The present search report has been drawn up for all claims

| Place of search | Date of completion of search | Examiner |
|---|---|---|
| Berlin | 08 February 91 | RUFET J.M.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document